# EUROPÄISCHE PATENTSCHRIFT

(11) **EP 0 774 930 B1**
(45) Veröffentlichungstag und Bekanntmachung des Hinweises auf die Patenterteilung: **22.03.2000**
(21) Anmeldenummer: 95926958.0
(22) Anmeldetag: 19.07.1995
(51) Int. Cl.: A61B 19/02, B65D 75/36

(54) **VERPACKUNG FÜR ZU STERILISIERENDE ARTIKEL**
PACKAGING FOR ARTICLES THAT ARE TO BE STERILIZED
EMBALLAGE POUR ARTICLES A STERILISER

(30) Priorität: 10.08.1994 DE 4428291
(43) Veröffentlichungstag der Anmeldung: 28.05.1997
(73) Patentinhaber: von Borries, Horst, 47839 Krefeld (DE)
(72) Erfinder: VON BORRIES, Horst, D-47839 Krefeld (DE); FONTEYNE, Gerard, B-9940 Evergem (BE)
(86) Internationale Anmeldenummer: EP9502850
(87) Internationale Veröffentlichungsnummer: WO9604861

(56) Entgegenhaltungen:
- EP-A- 0 056 323
- EP-A- 0 127 466
- EP-A- 0 437 856
- GB-A- 2 001 928
- GB-A- 2 070 514
- US-A- 4 810 541

## Beschreibung

Die Erfindung betrifft eine Verpackung für zu sterilisierende Artikel, bestehend aus einer gegebenenfalls tiefgezogenen, eine Haube bildenden Kunststoffolie und eine den Packungsboden bildenden Papierbahn, wobei die Papierbahn mit der Folie peelfähig versiegelt ist.

Verpackungen mit Sterilverpackungsgut sind beispielsweise aus der DE 8706916 U1 bekannt, der zu entnehmen ist, daß eine Kunststoffolienbahn mit tiefgezogenen Mulden mit einer Papierbahn peelfähig versiegelt wird, wobei die Papierbahn gasdurchlässig ist so, daß das Verpackungsgut nach dem Verpacken durch Druckbehandlung mit keimabtötenden Gas sterilisiert werden kann.

Aus der US 4810541-A ist ein versiegelbarer Lebensmittelbehälter bekannt, der mit einer Deckelfolie abgedeckt ist. Der Behälter als solches ist ein tiefgezogenes Plastikformteil aus einer mehrlagigen Folie. Die Innenschicht des Behälters ist dabei als Peelschicht ausgeführt und besteht aus einer Mischung von Polyethylen, Ethylenvinylacetatcopolymer und Butenpolymer. Die Deckelfolie ist eine beidseitig beschichtete Aluminiumfolie, die Außenlage besteht aus Polyester, die dem Verpackungsgut zugewandte Innenlage aus Polyethylen. Die Peelschicht wird beim Verschließen des Behälters mit der Polyethylenlage des Deckels versiegelt. Das Problem der Gassterilisation des Inhaltes ist nicht angesprochen.

Aus der DE 1927746 ist eine Verpackung für sterilisierte Artikel bekannt, die aus einem Kunststoffgefäß mit einer Öffnung besteht, wobei die Öffnung mit einem kunststoffimprägnierten, feuchtigkeitsdichten und keimfreien Papier abgedeckt ist. Das zur Papierimprägnierung eingesetzte Kunstharz ist ein Polyacryl, Polyester oder ein Polyvinylalkohol. Auf die Papierrückseite ist zusätzlich ein Schmelzkleber aus Polyethylen oder einem Mischpolymerisat, Polyethylenvinylacetat aufgetragen.

Die WO 8603976 beschreibt eine Sterilpackung die aus einem inneren und einem äußeren Umschlag besteht. Der äußere Umschlag gestattet eine Gassterilisation des inneren Umschlages und dessen Inhaltes, im Anschluß daran wird der äußere Umschlag versiegelt so, daß der Inhalt bakterienfrei bleibt.

Der große Nachteil dieser und ähnlicher Konstruktionen nach dem Stande der Technik liegt darin, daß beim Öffnen der Packungen das im allgemeinen nach dem Peelverfahren geschieht, also dadurch, daß die Kunststoffolie von der Papierbahn abgezogen wird, die Kunststoffolie Fasern aus der Papierbahn herausreißt und dadurch das verpackte sterile Gut verunreinigt wird. Nach den Konstruktionen des Standes der Technik ist es also nicht möglich eine Sterilpackung, die aus Papier und Kunststoffolie besteht, so zu öffnen, daß keine Fasern und/oder Faserbruchstücke dabei freigesetzt werden.

Aufgabe der Erfindung ist daher das Öffnen einer gassterilisierbaren Verpackung so zu ermöglichen, daß keine Fasern vom Papier abgerissen werden, die Öffnung also völlig faserfrei erfolgt, obwohl die Folie nach dem Peelverfahren abgezogen wird.

Gelöst wird diese Aufgabe bei einer Verpackung für zu sterilisierende Artikel die aus einer, gegebenenfalls tiefgezogenen, eine Haube bildenden Kunststoffolie und einer den Packungsboden bildenden Papierbahn besteht, wobei die Papierbahn mit der Folie peelfähig versiegelt ist, dadurch daß die Kunststoff-Folie (4) eine Mehrschichtfolie ist deren dem Papier (6) zugewandte Seite eine Zusammensetzung aus mindestens einem
a) Äthylenpolymeren und/oder einem Äthylen-Vinylacetatcopolymeren, sowie
b) einem Styrolhomopolymer, und/oder
c) einem kautschukartigem Polymer ist;
wobei
a) in einer Menge von 55 - 95 Gewichtsprozent als Polyäthylenpolymer mit einer Dichte von 0,91 bis 0,93 g/ccm und einem Schmelzindex von 0,5 bis 7,0 g/10min vorliegt und das Äthylen-Vinyl-Acetat-Copolymer maximal 10 Gewichtsprozent Vinylacetat aufweist;
b) das Styrolhomopolymer in einer Menge von 0 bis 30 Gewichtsprozent vorhanden ist;
c) ein elastomeres thermoplastisches Styrol-Butadien-Styrol-Blockcopolymer oder Styrol-Isopren-Styrol-Blockcopolymer in einer Menge von 0 bis 20 Gewichtsprozent und/oder ein Polybutylen- oder Polyisobutylenpolymer in einer Menge von 0 bis 30 Gewichtsprozent vorhanden ist;
das Papier des Packungsbodens ein mit Kunststoff behandeltes Papier ist, das eine mittlere Gasdurchlässigkeit von mindestens 0,50 µm/Pa.s aufweist; und die Kunststoff-Folie mit der Papierbahn versiegelt ist.

Bevorzugt liegt das Flächengewicht des Papieres zwischen 55 und 250 g/m², das Gewicht des Kunststoffauftrags liegt zwischen 3 und 20 g/m², der Kunststoff ist vorzugsweise ein EVA - Copolymer.

Das Kunststofflaminat wird zweckmäßig durch Koextrusion hergestellt, kann aber auch durch Klebekaschieren mit einer Mono- oder Koextrudierten Folie gefertigt werden. Es weist bevorzugt einen fünfschichtigen Aufbau auf, daß heißt, über der dem Papier zugekehrten Peelschicht ist eine Haftvermittlerschicht angeordnet die eine Polyamidschicht mit der Peelschicht verbindet und ihrerseits durch eine weitere Haftvermittlerschicht mit einer Polyäthylenschicht verbunden ist.

Die Polyamidschicht kann zweckmäßig auch gegen eine Polystyrolschicht - Homo- oder Copolymere - ausgetauscht werden, in diesen Fällen dient die Kombination Polyäthylen/Polyamid bzw. Polyäthylen/Polystyrol, als stabilisierender Bestandteil für die thermoplastiche Verformung, das heißt für den Tiefziehvorgang.

Ferner ist es möglich statt Polyamid HD- oder ND-Polyäthylen sowie Copolymere ( EVA, LLDPE, EBA, EMA, EAA usw. ) zu verwenden. Bewährt haben sich auch Folien aus Polycarbonat, Polyester und Copolyester sowie Polyvinylchlorid.

Der zwischen den einzelnen Schichten angeordnete Haftvermittler ist vorzugsweise ein EVA-Copolymer oder ein EAA-Terpolymer.

Gemäß einer weiteren zweckmäßigen Ausgestaltung der Erfindung, besteht das Laminat aus der Peelschicht die über einen Kaschierkleber mit einer LDPE-Folie und darauf kaschierter HDPE-Folie verbunden ist. Auch diese Folienkombination ist gut thermoplastich verformbar und weist eine gute Formstabilität auf.

Der Kunststoff, der zweckmäßig nur einseitig auf das Papier aufgebracht ist, ist bevorzugt ein EVA-Copolymer, er erstreckt sich gemäß einer besonders bevorzugten Ausführungsform der Erfindung, im wesentlichen über die gesamte Papierstärke, das heißt, daß der Kunststoff beim Aufbringen auf das Papier dieses im wesentlichen durchdringt, es erfolgt dadurch eine feste Einbindung der Fasern, ohne daß die Porosität wesentlich verringert wird.

Vorteilhaft besteht das Papier aus einer groben Zellstoffsorte, die, gemäß einer weiteren zweckmäßigen Ausgestaltung der Erfindung, nicht oder nur unwesentlich gemahlen ist. In Verbindung mit dem weiteren vorteilhaften Merkmal, daß das Papier ein ungefülltes Papier ist, also keine Pigmente enthält, wird die angestrebte hohe Luftdurchlässigkeit sichergestellt und gleichzeitig eine hohe Festigkeit erreicht.

Diese Festigkeit wird gemäß einer bevorzugten Ausgestaltung der Erfindung noch dadurch gesteigert, daß das Papier mit Natriumwasserglas verfestigt wird.

Eine sehr vorteilhafte Ausgestaltung der Erfindung sieht vor, daß die Kunststofffolie gegebenenfalls eine Mehrschichtfolie ist, deren dem Papier zugewandte Seite ein Polypropylen oder ein Polypropylencopolymerisat ist; das Papier des Packungsbodens ein mit Kunststoff behandeltes Papier ist, das eine mittlere Gasdurchlässigkeit von mindestens 0,50 µm/Pa.s aufweist, ein Flächengewicht zwischen 55 und 250 g/m² besitzt, der Kunststoffauftrag ein Gewicht von 3 - 20 g/m² aufweist, der Kunststoff ein EVA-Copolymer ist, das einseitig auf das Papier (6) aufgebracht ist und sich im wesentlichen über die gesamte Papierstärke erstreckt.

### Beispiel 1

Auf einem handelsüblichen Form- und Packautomaten Fabrikat Tiromat Modell 3000 wurden Sterilpackungen für das Verpacken von Einwegspritzen gefertigt. Die einzelne Sterilpackung wies eine Größe von 44 x 125 mm auf, sie bestandt aus einer keulenförmige Mulde, die bei einer Länge von ca 100 mm am dickeren Ende einen Radius von 15 mm und am dünneren Ende einen Radius von 5 mm aufwies. Als Folie wurde eine handelsübliche Polyamid-Polyäthylen-Verbundfolie mit einer Stärke von 95 µm eingesetzt. Die Rollenbreite betrug 465 mm, die Rollenlänge 900 m. Auf einer Rollenbreite konnten dabei 10 Packungen untergebracht werden. Die Machinengeschwindigkeit betrug 300 Packungen pro Minute. Die Formtemperatur für die Folie 115 °C.

Das Tiefziehen der Mulden erfolgte mit Druckluft von 5 bar. Nach dem Einbringen der Einwegspritzen wurde über die PA-PE-Verbundfolie ein handelsübliches medizinisches Papier geführt, angedrückt und im Konturbereich der Stege mit der Kunststoffolie versiegelt. Die Siegeltemperatur lag bei 180 ° C, die Siegelzeit bei 1 Sekunde. Das medizinische Papier wies ein Gewicht von 60g/cm² auf. Beim Siegeln wurde eine feste und dichte Verbindung zwischen der Verbundfolie und dem medizinischen Papier erreicht. Nach Trennen der gefertigten Sterilpackungen in Einzelpackungen wurden sie einer Sterilisation von 7 Stunden bei einer Temperatur von 45 - 50°C und 50 bis 60% Luftfeuchtigkeit in einem Auto-klaven von 40 Kubikmetern Fassungsvermögen ausgesetzt. Als Sterilisationsmedium diente Äthylenoxydgas in einer Konzentration von 800 mg/l. Die in einigen Packungen angeordneten Sterilisationsmarken zeigten an, daß die Sterilisation erfolgreich stattgefunden hatte.

Beim Öffnen einzelner Packungen ergab sich, daß aus dem medizinischen Papier Fasern herausgerissen worden waren, die sich im Schweißnahtbereich befunden hatten, ein Teil dieser Fasern und Faserbruchstücke war lose und fiel auf die Einwegspritze, andere Fasern hingen mehr oder weniger fest an der Siegelschicht der Mehrschichtfolie.

### Beispiel 2

Abweichend von Beispiel 1 wurde statt eines handelsüblichen medizinischen Papieres und der handelsüblichen Verbundfolie, das Papier und die Folie gemäß der Erfindung eingesetzt. Die Kunststoffolie war dabei als fünfschichtiges Laminat ausgeführt, mit einer Außenschicht aus Polyamid, einer darauf aufgebrachten Haftschicht darauf einer Polyäthylenschicht, darauf einer Haftschicht und, auf der dem Papier zugewandten Seite, einer Peelschicht. Beide Haftschichten bestanden aus einem Äthylen-Vinylacetat-Copolymer. Die Peelschicht wies folgende Zusammensetzung auf: 70 Gewichtsprozent Polyäthylen, mit einer Dichte von 0.922 g/ccm, einem Schmelzindex von 0,85 g/lOmin, 20 Gewichtsprozent Sty-rolhomopolymer und 10 Gewichtsprozent Styrol-Butadien-Styrolblockcopolymer.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst worden waren. Im Siegelbereich war deutlich eine " Spur " in Form einer weißen Linie auf der Peelfolie zu erkennen, d.h. sichtbar, daß die Packung geöffnet worden war.

### Beispiel 3

Die Sterilpackung wurde analog Beispiel 1 gefertigt, als Kunststoffolie wurde jedoch ein fünfschichtiges Material analog Beispiel 2 eingesetzt. Die-Peelschicht wies folgende Zusammensetzung auf 90 Gewichtsprozent Polyäthylen, mit einer Dichte von 0,922 g/ccm und einem Schmelzindex von 0,85g/10min, sowie 10 Gewichtsprozent Styrolhomopolymer.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden.

### Beispiel 4

Die Sterilpackung wurde analog Beispiel 1 gefertigt, als Kunststoffolie wurde jedoch ein fünfschichtiges Material analog Beispiel 2 eingesetzt. Die Peelschicht wies folgende Zusammensetzung auf: 70 Gewichtsprozent Polyäthylen, mit einer Dichte von 0,944 g/ ccm und einem Schmelzindex von 0,17 g/10min, 20 Gewichtsprozent Styrolhomopolymer und 10 Gewichtsprozent Styrol-Butadien-Styrolblockcopolymer.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden. Im Siegelbereich war deutlich eine " Spur " in Form einer weißen Linieauf der Peelfolie zu erkennen, d.h. sichtbar, daß die Packung geöffnet worden war.

### Beispiel 5

Die Sterilpackung wurde analog Beispiel 1 gefertigt, als Kunststoffolie wurde jedoch ein fünfschichtiges Material analog Beispiel 2 eingesetzt. Die Peelschicht wies folgende Zusammensetzung auf: 65 Gewichtsprozent Polyäthylen, mit einer Dichte von 0,928 g/ccm und einem Schmelzindex von 1,6g/10min ÄthylenVinylacetatcopolymer mit 4 Gewichtsprozent Vinylacetat, 20 Gewichtsprozent Styrolhomopolymer und 15 Gewichtsprozent Styrol-Butadien-Styrolblockcopolymer.

Das Papier wies ein Flächengewicht von 104,5 g/m² auf, das Gewicht des Kunststoffauftrags lag bei 3,5 g/m², als Kunststoff wurde ein EVA-Copolymer aufgebracht, die mittlere Gasdurchlässigkeit des Papieres betrug 1,2 µm /Pa s.

Die Sterilisation erfolgte analog Beispiel 1, das Aufreißen der Packungen ergab, daß keine Papierfasern vom Papier gelöst wurden. Im Siegelbereich war deutlich eine " Spur " in Form einer weißen Linie auf der Peelfolie zu erkennen, d.h. sichtbar, daß die Packung geöffnet worden war.

### Beispiel 6

Die Sterilpackung wurde analog Beispiel 1 gefertigt, als Kunststoffolie wurde jedoch ein fünfschichtiges Material analog Beispiel 2 eingesetzt. Die Peelschicht wies folgende Zusammensetzung auf: 68 Gewichtsprozent Polyäthylen, mit einer Dichte von 0,923g/ccm und einem Schmelzindex von 0,3g/10min, 20 Gewichtsprozent Styrolhomopolymer und 12 Gewichtsprozent Styrol-Isopren-Styrolblockcopolymer.

Das Papier wies ein Flächengewicht von 104,5 g/m² auf, das Gewicht des Kunststoffauftrags lag bei 3,5 g/m², als Kunststoff wurde ein EVA-Copolymer aufgebracht, die mittlere Gasdurchlässigkeit des Papieres betrug 1,2 µm /Pa s.

Die Sterilisation erfolgte analog Beispiel 1, das Aufreißen der Packungen ergab, daß keine Papierfasern vom Papier gelöst wurden. Im Siegelbereich war deutlich eine " Spur " in Form einer weißen Linie auf der Peelfolie zu erkennen, d.h. sichtbar, daß die Packung geöffnet worden war.

### Beispiel 7

Bei einem analogen Versuchsaufbau wie in Beispiel 2 wurde jedoch eine dreischichtige Folie eingesetzt deren Außenschicht aus HDPE-Folie, die Mittelschicht aus LDPE-Folie und die Peelschicht aus 70 Gewichtsprozent Polyäthylen, mit einer Dichte von 0,944 g/ccm und einem Schmelzindex von 0,17g/10min 20 Gewichtsprozent Styrolhomopolymer und 10 Gewichtsprozent Styrol-Butadien-Styrolblockcopolymer bestand.Die Folie wurde nicht tiefgezogen, es wurden Steriltücher in Flachbeutel abgepackt.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden. Im Siegelbereich war deutlich eine " Spur " in Form einer weißen Linie auf der Peelfolie zu erkennen, d.h. sichtbar, daß die Packung geöffnet worden war.

### Beispiel 8

Die Sterilpackung wurde analog Beispiel 1 gefertigt, als Kunststoffolie wurde jedoch ein fünfschichtiges Material analog Beispiel 2 eingesetzt. Die Peelschicht wies folgende Zusammensetzung auf: 80 Gewichtsprozent Polyäthylen, mit einer Dichte von 0,922 g/ccm und einem Schmelzindex von 0,85g/10min, sowie 20 Gewichtsprozent Polybutenpolymer.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden.

### Beispiel 9

Die Sterilpackung wurde analog Beispiel 1 gefertigt, als Kunststoffolie wurde jedoch ein fünfschichtiges Material analog Beispiel 2 eingesetzt. Die Peelschicht wies folgende Zusammensetzung auf: 95 Gewichtsprozent Polyäthylen, mit einer Dichte von 0,923 g/ccm und einem Schmelzindex von 0,86g/10min, sowie 5 Gewichtsprozent Polybutenpolymer.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab: daß keine Papierfasern vom Papier gelöst wurden.

### Beispiel 10

Die Sterilpackung wurde analog Beispiel 1 gefertigt, als Kunststoffolie wurde jedoch ein fünfschichtiges Material analog Beispiel 2 eingesetzt. Die Peelschicht wies folgende Zusammensetzung auf: 90 Gewichtsprozent Polyäthylen, mit einer Dichte von 0,925 g/ccm und einem Schmelzindex von 2,0g/10min, sowie 10 Gewichtsprozent Polyisobutylen.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden.

### Beispiel 11

Die Sterilpackung wurde analog Beispiel 1 gefertigt, als Kunststoffolie wurde jedoch ein fünfschichtiges Material analog Beispiel 2 eingesetzt. Die Peelschicht wies folgende Zusammensetzung auf: 45 Gewichtsprozent Polyäthylen, mit einer Dichte von 0,922 g/ccm und einem Schmelzindex von 0,85 g/10min, 45 Gewichtsprozent Äthylenvinyl-acetatcopolymer mit 4 Gewichtsprozent Vinylacetat, mit einer Dichte von 0,925 g/ccm und einem Schmelzindex von 0,5g/10min sowie 15 Gewichtsprozent Polybuten.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden. Im Siegelbereich war deutlich eine " Spur " in Form einer weißen Linie auf der Peelfolie zu erkennen, d.h. sichtbar, daß die Packung geöffnet worden war.

### Beispiel 12

Die Sterilpackung wurde analog Beispiel 1 gefertigt, als Kunststoffolie wurde jedoch ein fünfschichtiges Material analog Beispiel 2 eingesetzt. Die Peelschicht wies folgende Zusammensetzung auf: 70 Gewichtsprozent Polyäthylen, mit einer Dichte von 0,923g/ccm und einem Schmelzindex von 0,85g/10min, 20 Gewichtsprozent Styrolhomopolymer und 10 Gewichtsprozent Styrol-Butadien-Styrolblockcopolymer.

Das Papier wies ein Flächengewicht von 62,5 g/m² auf, das Gewicht des Kunststoffauftrags lag bei 3 g/m², als Kunststoff wurde ein EEA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden. Im Siegelbereich war deutlich eine " Spur " in Form einer weißen Linie auf der Peelfolie zu erkennen, d.h. sichtbar, daß die Packung geöffnet worden war.

### Beispiel 13

Die Sterilpackung wurde analog Beispiel 1 gefertigt, als Kunststoffolie wurde jedoch ein fünfschichtiges Material analog Beispiel 2 eingesetzt. Die Peelschicht wies folgende Zusammensetzung auf: 70 Gewichtsprozent Polyäthylen, mit einer Dichte von 0,923g/ccm und einem Schmelzindex von 0,85g/10min, 20 Gewichtsprozent Styrolhomopolymer und 10 Gewichtsprozent Styrol-Butadien-Styrolblockcopolymer.

Das Papier wies ein Flächengewicht von 62,5 g/m² auf, das Gewicht des Kunststoffauftrags lag bei 3 g/m², als Kunststoff wurde ein EVA-Copolymer aufgebracht, des weiteren 2,5Gewichtsprozent Natriumwasserglas, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden. Im Siegelbereich war deutlich eine " Spur " in Form einer weißen Linie auf der Peelfolie zu erkennen, d.h. sichtbar, daß die Packung geöffnet worden war.

### Beispiel 14

Bei einem anlogen Versuchsaufbau wie in Beispiel 2 wurde eine mehrschichtige Folie eingesetzt, deren Außenschicht aus einer 12 µm starken, biorientierten Polyesterfolie bestand, die mittels Klebekaschierung mit einer koextrudierten Peelfolie verbunden war. Die Peelfolie bestand aus drei Schichten, einer plasmabehandelten LDPE-Schicht, einer Haftschicht auf Basis von Polyäthylen oder Äthylenvinylacetatcopolymer und der Peelschicht mit folgender Zusammensetzung: 70 Gewichtsprozent Polyäthylen, mit einer Dichte von 0,944 und einem Schmelzindex von 0,17g/10min, 20 Gewichtsprozent Styrolhomopolymer und 10 Gewichtsprozent Styrol-Butadien-Styrolblockcopolymer. Die Mehrschichtfolie wurde nicht tiefgezogen sondern zu Flachbeuteln verarbeitet.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden. Im Siegelbereich war deutlich eine " Spur " in Form einer weißen Linie auf der Peelfolie zu erkennen, d.h. sichtbar, daß die Packung geöffnet worden war.

### Beispiel 15

Abweichend von Beispiel 1 wurde statt eines handelsüblichen medizinischen Papieres und der handelsüblichen Verbundfolie, das Papier und die Folie gemäß der Erfindung eingesetzt. Die Kunststoffolie war dabei als fünfschichtiges Laminat ausgeführt, mit einer Außenschicht aus Polyamid, einer darauf aufgebrachten Haftschicht, darauf einer Polyäthylenschicht, darauf einer Haftschicht und, auf der dem Papier zugewandten Seite, einer Polypropylenschicht. Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.
Die Sterilisation erfolgte analog Beispiel 1, das Aufreißen der Packungen ergab, daß keine Papierfasern vom Papier gelöst worden waren.

### Beispiel 16

Die Sterilpackung wurde analog Beispiel 1 gefertigt, als Kunststoffolie wurde jedoch ein fünfschichtiges Material analog Beispiel 2 eingesetzt. Die Peelschicht bestand aus einem Polypropylencopolymerisat. Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden.

### Beispiel 17

Bei einem analogen Versuchsaufbau wie in Beispiel 2 wurde jedoch eine dreischichtige Folie eingesetzt deren Außenschicht aus HDPE-Folie, die Mittelschicht aus LDPE-Folie und die Peelschicht aus Polypropylen, mit einer Dichte von 0,905 g/ccm und einem Schmelzindex von 2,00g/10min bestand.Die Folie wurde nicht tiefgezogen, es wurden Steriltücher in Flachbeutel abgepackt.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden.

### Beispiel 18

Die Sterilpackung wurde analog Beispiel 1 gefertigt, als Kunststoffolie wurde jedoch eine einschichtige Polypropylenfolie in einer Stärke von 40µm, mit einer Dichte von 0,905 g/ccm und einem Schmelzindex von 4,00g/10min eingesetzt.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden.

### Beispiel 19

Die Sterilpackung wurde analog Beispiel 1 gefertigt, als Kunststoffolie wurde jedoch ein einschichtiges Material, Polypropylencopolymer eingesetzt, das folgende Daten aufwies: Stärke 25µm, Dichte von 0,900 und einen Schmelzindex von 6,00g/10min.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden.

### Beispiel 20

Bei einem anlogen Versuchsaufbau wie in Beispiel 2 wurde eine mehrschichtige Folie eingesetzt, deren Außenschicht aus einer 12 µm starken, biorientierten Polyesterfolie bestand, die mittels Klebekaschierung mit einer koextrudierten Peelfolie verbunden war. Die Peelfolie bestand aus drei Schichten, einer plasmabehandelten LDPE-Schicht, einer Haftschicht auf Basis von Polyäthylen- oder Äthylenvinylacetatcopolymer und als Peelschicht Polypropylen in einer Stärke vo 20 µm, mit einer Dichte von 0,905 und einem Schmelzindex von 18,00g/10min.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst wurden.

### Beispiel 21

Abweichend von Beispiel 1 wurde statt eines handelsüblichen medizinischen Papieres und der handelsüblichen Verbundfolie, das Papier und die Folie gemäß der Erfindung eingesetzt. Die Kunststoffolie war dabei als fünfschichtiges Laminat ausgeführt, mit einer Außenschicht aus Polyamid, einer darauf aufgebrachten Haftschicht darauf einer Polyäthylenschicht, darauf einer Haftschicht und, auf der dem Papier zugewandten Seite, einer Peelschicht. Beide Haftschichten bestanden aus einem Äthylen-Vinylacetat-Copolymer. Die Peelschicht wies folgende Zusammensetzung auf: 67 Gewichtsprozent Polyäthylen, mit einer Dichte von 0.922 g/ccm, einem Schmelzindex von 0,85 g/10min, 3 Gewichtsprozent eines Masterbatches mit einer Kontrastfarbe - blau -, 20 Gewichtsprozent Sty-rolhomopolymer und 10 Gewichtsprozent Styrol-Butadien-Styrolblockcopolymer.

Das Papier wies ein Flächengewicht von 62,5 g/m² auf, das Gewicht des Kunststoffauftrags lag bei 3 g/m², als Kunststoff wurde ein EVA-Copolymer aufgebracht, die mittlere Gasdurchlässigkeit des Papieres betrug 2,2 µm /Pa s.

Die Sterilisation erfolgte analog Beispiel 1, das Aufreißen der Packungen ergab, daß keine Papierfasern vom Papier gelöst worden waren. Im Siegelbereich war auf dem Papier deutlich eine blaue " Spur " als Linie zu erkennen, d.h. sichtbar, daß die Packung geöffnet worden war.

Die Erfindung wird nachstehend anhand der Zeichnungen beschrieben.

Es zeigt
- Figur 1: die Draufsicht auf eine Sterilpackung
- Figur 2: die Vorderansicht im Schnitt einer Sterilpac kung
- Figur 3: die Seitenansicht bei teilgeöffneter Packung
- Figur 4 bis Figur 7: REM - Aufnahmen des Papieres
- Figur 8: ein fünfschichtiges Laminat im Schnitt
- Figur 9: ein vierschichtiges Laminat im Schnitt
- Figur 10: ein zweischichtiges Laminat im Schnitt
- Figur 11: ein fünfschichtiges Laminat im Schnitt
- Figur 12: ein dreischichtiges Laminat im Schnitt
- Figur 13: eine Monofolie im Schnitt
- Figur 14: eine Monofolie im Schnitt

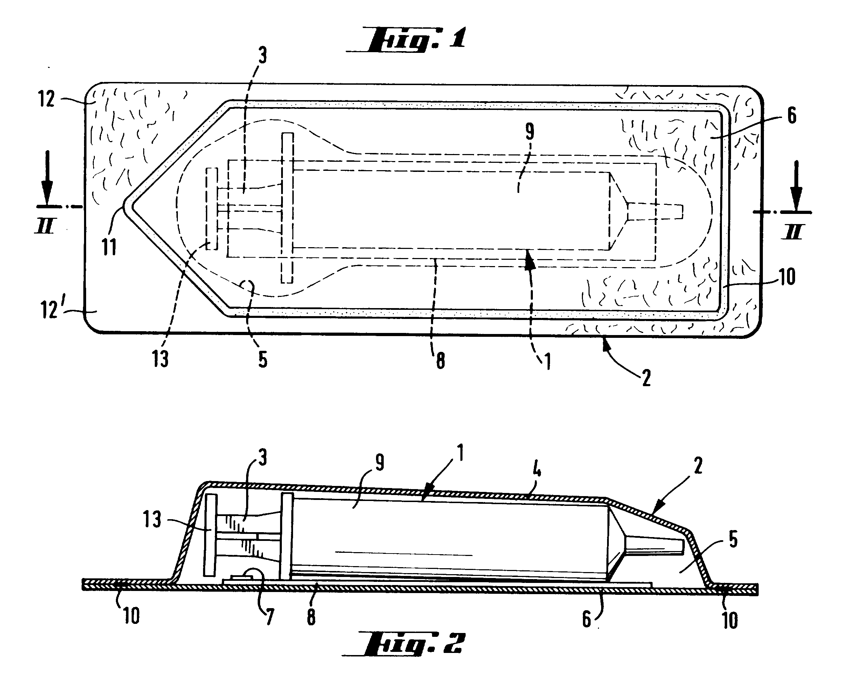

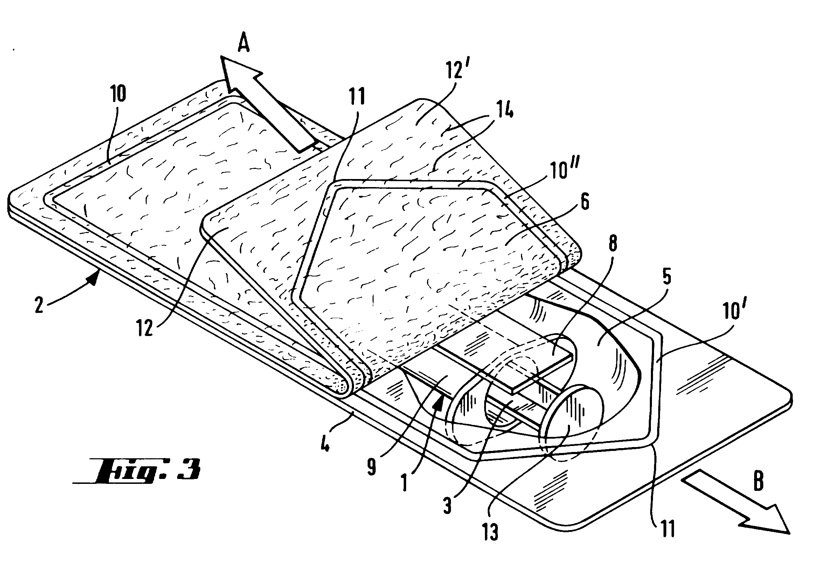

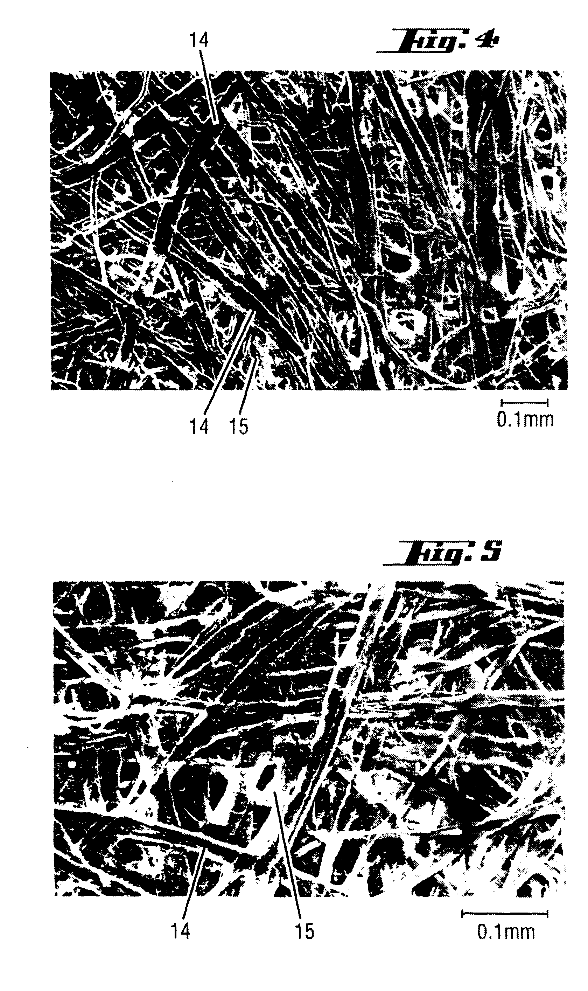

Die Blisterpackung 2 weist, wie in den Figuren 1 und 2 dargestellt, den Aufnahmeraum 5 auf, der durch Tiefziehen der Folienbahn 4 in diesem Bereich entstanden ist. Der Aufnahmeraum 5 nimmt die Einwegspritze 1 auf, die im wesentlichen aus dem Kolben 3 und dem Zylinder 9 besteht. Abgedeckt wird der Aufnahmeraum 5 durch die Papierbahn 6, die entlang der Siegelnaht 10 mit der Folienbahn 4 verschweißt ist. Die Schweißnaht 10 weist im linken Teil der Blisterpackung 2 eine Ecke 11 auf von der aus die Blisterpackung 2 mittels der Lappen 12 bzw.12', also der Bereiche an denen die Folienbahn 4 nur lose auf der Papierbahn 6 aufliegt, aufgerissen werden kann. Wie in Figur 2 dargestellt, enthält die Blisterpackung 2 außer der Einwegspritze 1 noch eine Einlage mit Anweisungen, auf die gleichzeitig die Sterilmarke 7 aufgebracht ist. Nach dem Tiefziehen der Folienbahn 4 wird also zunächst die Einwegspritze 1 in den gebildeten Aufnahmeraum 5 eingelegt, darüber die Einlage 8 mit der Anweisung und der darauf aufgebrachten Sterilmarke 7 eingefügt und die Folienbahn 4, also die nebeneinander angeordneten gefüllten Aufnahmeräume 5, erst danach mit der Papierbahn 6 abgedeckt und verschweißt.

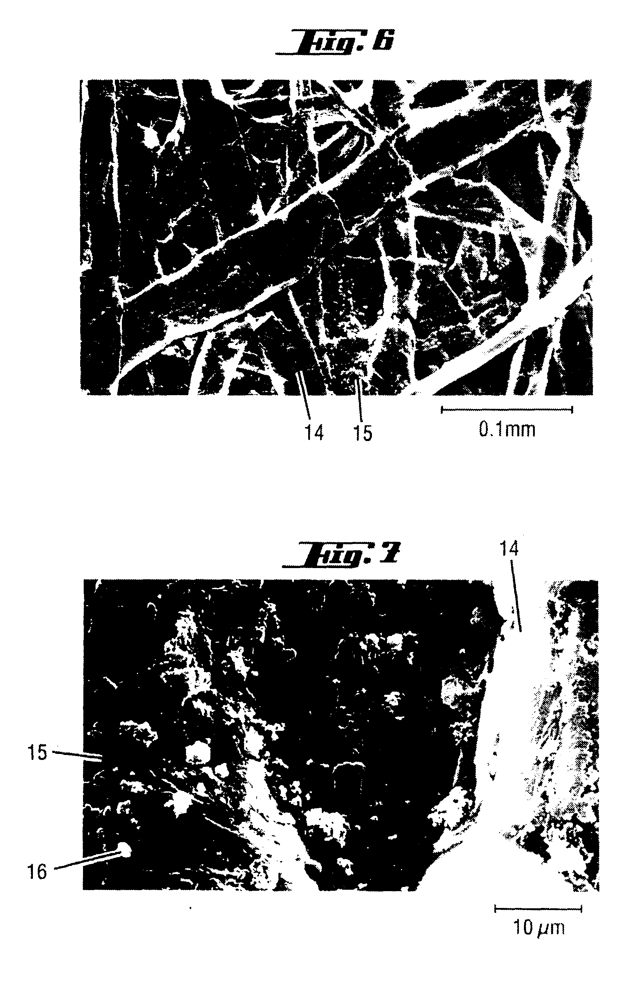

Wie Figur 3 zeigt, wird zum Öffnen der Sterilpackung 2 die Folienbahn 4 und die Papierbahn 6 im Bereich der Ecke 11 der Schweißnaht 10 an den Lappen 12, 12' erfaßt und in Richtung der Pfeile A bzw. B auseinander gezogen. Der Druckkopf 13 der Einwegspritze 1 wird dadurch freigelegt und kann ergriffen werden um die Einwegspritze aus der Packung zu entnehmen.

Wie der Figur 3 klar zu entnehmen, weist die Papierbahn 6 über die gesamte Fläche verteilt, also auch im Bereich der Siegelnaht 10", Papierfasern 14 auf, wohingegen die Siegelnaht 10', die sich auf der Folienseite befindet, keinerlei Spuren von anhaftenden Fasern 14 aufweist.

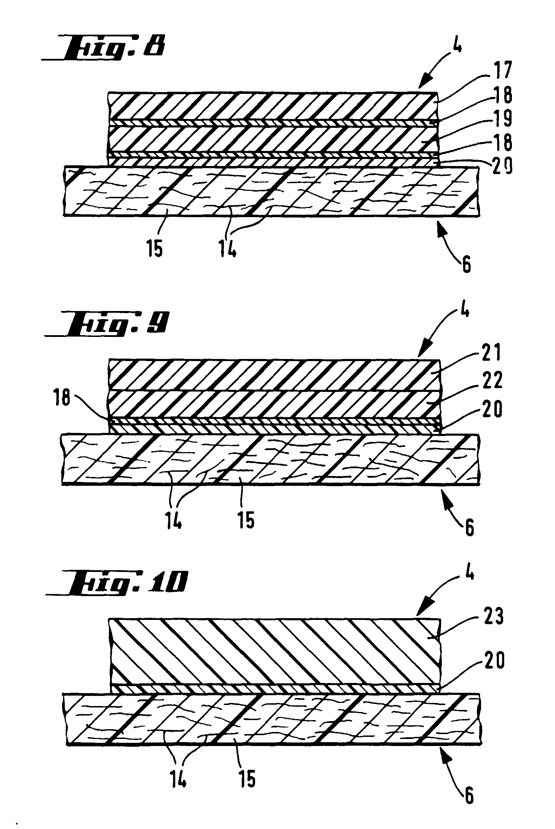

Die Figuren 4 - 7 zeigen, daß die Fasern 14 zumindest in Teilbereichen von Kunststoff 15 umhüllt sind. Dieser Kunststoff ist ein EVA-Coplymer. Figur 7 zeigt zusätzlich dazu Natriumwasserglaspartikel 16 die zwischen den Fasern 14 verteilt angeordnet sind.

Die Figuren 8 - 10 zeigen in starker Vergrößerung einen Ausschnitt - im Querschnitt - aus einer gesiegelten Packung. Figur 8, die Beispiel 2 entspricht, zeigt in der Außenschicht eine Polyamidfolie 17 die über eine Kaschierung mit Äthylen-Vinylacetat 18 mit einer Polyäthylenfolie 19 verbunden ist. Auf diese ist, ebenfalls über eine EVA-Kaschierung 18 eine Peelfolienschicht 20 aufgebracht.

Figur 9, die Beispiel 7 entspricht, zeigt als Außenschicht eine hd-Polyäthylenfolie 21, die durch Coextrusion mit einer ld-Polyäthylenfolie 22 erzeugt wurde. Auf diese ist über eine EVA-Kaschierung 18 eine Peelfolienschicht 20 aufgebracht.

Figur 10 zeigt als Außenschicht eine Polypropylenfolie 23, die durch Coextrusion mit einer Peelfolie 20 erzeugt wurde.

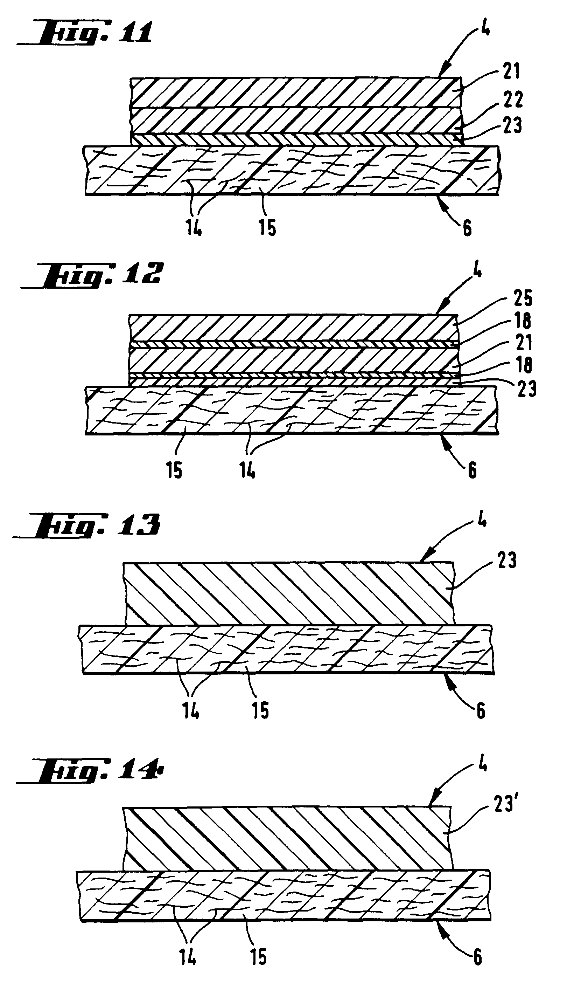

Die Figuren 11 - 13 zeigen Folien bei denen die Siegelschicht von einer Polypropylenfolie 23 gebildet wird.

Figur 11 entspricht dabei Beispiel 17, die Außenschicht besteht aus HDPE-Folie 21, die Mittelschicht aus LDPE-Folie 22 und die Peelschicht aus Polypropylen 23.

Figur 12 entspricht Beispiel 20, die Außenschicht besteht aus einer 12 µm starken, biorientierten Polyesterfolie 25, die mittels Klebekaschierung 18 mit einer koextrudierten Peelfolie verbunden ist. Die Peelfolie besteht aus drei Schichten, einer plasmabehandelten LDPE-Schicht 21, einer Haftschicht auf Basis Äthylenvinylacetatcopolymer 18 und als Peelschicht Polypropylen 23 in einer Stärke vo 20 µm

Figur 13 entspricht Beispiel 18, eine einschichtige Polypropylenfolie 23 in einer Stärke von 40µm bildet die Peelfolie.

Figur 14 entspricht Beispiel 19, ein einschichtiges Material, Polypropylencopolymer 23' bildet die Peelfolie. Alle Folien sind gegen die Papierbahn 6 gesiegelt.

## Patentansprüche

1. Verpackung für zu sterilisierende Artikel bestehend aus einer, gegebenenfalls tiefgezogenen, eine Haube bildenden Kunststoff-Folie und einer den Packungsboden bildenden Papierbahn, wobei die Papierbahn mit der Folie peelfähig versiegelt ist,
dadurch gekennzeichnet,
daß die Kunststoff-Folie (4) eine Mehrschichtfolie ist, deren dem Papier (6) zugewandte Seite eine Zusammensetzung aus mindestens einem
a) Äthylenpolymeren und/oder einem Äthylen-Vinylacetatcopolymeren, sowie
b) einem Styrolhomopolymer, und/oder
c) einem kautschukartigem Polymer ist;
wobei
a) in einer Menge von 55 - 95 Gewichtsprozent als Polyäthylenpolymer mit einer Dichte von 0,91 bis 0,93 g/ccm und einem Schmelzindex von 0,5 bis 7,0 g/10min vorliegt und das Äthylen-Vinyl-Acetat-Copolymer maximal 10 Gewichtsprozent Vinylacetat aufweist;
b) das Styrolhomopolymer in einer Menge von 0 bis 30 Gewichtsprozent vorhanden ist;
c) ein elastomeres thermoplastisches Styrol-Butadien-Styrol-Blockcopolymer oder Styrol-Isopren-Styrol-Blockcopolymer in einer Menge von 0 bis 20 Gewichtsprozent und/oder ein Polybutylen- oder Polyisobutylenpolymer in einer Menge von o bis 30 Gewichtsprozent vorhandenist;
das Papier des Packungsbodens ein mit Kunststoff behandeltes Papier ist, das eine mittlere Gasdurchlässigkeit von mindestens 0,50 µm/Pa.s aufweist; und die Kunststoff-Folie mit der Papierbahn versiegelt ist.

2. Verpackung nach Anspruch 1, dadurch gekennzeichnet, daß die mehrschichtige Kunststoffolie (4) ein Coextrudat ist.

3. Verpackung nach Anspruch 1, dadurch gekennzeichnet, daß die mehrschichtige Kunststoffolie (4) ein Laminat ist.

4. Verpackung nach einem der Ansprüche 1 bis 3 dadurch gekennzeichnet, daß das Papier (6) ein Flächengewicht zwischen 55 und 250 g/m² aufweist.

5. Verpackung nach einem der Ansprüche 1 bis 4 dadurch gekennzeichnet, daß der Kunststoffauftrag ein Gewicht von 3 - 20 g/m² aufweist.

6. Verpackung nach einem der Ansprüche 1 bis 5 dadurch gekennzeichnet, daß der Kunststoff ein EVA-Copolymer ist.

7. Verpackung nach einem der Ansprüche 1 bis 6 dadurch gekennzeichnet, daß der Kunststoff einseitig auf das Papier (6) aufgebracht ist.

8. Verpackung nach einem der Ansprüche 1 bis 7 dadurch gekennzeichnet, daß der Kunststoff sich im wesentlichen über die gesamte Papierstärke erstreckt.

9. Verpackung nach einem der Ansprüche 1 bis 8 dadurch gekennzeichnet, daß das Papier (6) aus einer groben Zellstoffsorte besteht.

10. Verpackung nach einem der Ansprüche 1 bis 9 dadurch gekennzeichnet, daß der Zellstoff nicht oder nur unwesentlich gemahlen ist.

11. Verpackung nach einem der Ansprüche 1 bis 10 dadurch gekennzeichnet, daß das Papier (6) ein ungefülltes Papier ist.

12. Verpackung nach einem der Ansprüche 1 bis 11, dadurch gekennzeichnet, daß das Papier (6) mit Na-Wasserglas verfestigt wurde.

13. Verpackung für zu sterilisierende Artikel bestehend aus einer, gegebenenfalls tiefgezogenen, eine Haube bildenden Kunststoff-Folie und einer den Packungsboden bildenden Papierbahn, wobei die Papierbahn mit der Folie peelfähig versiegelt ist,
dadurch gekennzeichnet,
daß die Kunststofffolie (4) gegebenenfalls eine Mehrschichtfolie ist, deren dem Papier (6) zugewandte Seite ein Polypropylen oder ein Polypropylencopolymerisat ist; das Papier (6) des Packungsbodens ein mit Kunststoff behandeltes Papier ist, das eine mittlere Gasdurchlässigkeit von mindestens 0,50 µm/Pa.s aufweist, ein Flächengewicht zwischen 55 und 250 g/m² besitzt, der Kunststoffauftrag ein Gewicht von 3 - 20 g/m² aufweist, der Kunststoff ein EVA-Copolymer ist, das einseitig auf das Papier (6) aufgebracht ist und sich im wesentlichen über die gesamte Papierstärke erstreckt.

## Revendications

1. Emballage pour articles à stériliser, constitué d'une feuille de matière plastique formant une calotte, éventuellement emboutie, et d'une bande de papier formant le fond de l'emballage, la bande de papier étant scellée à la feuille de manière à être séparable, **caractérisé** en ce que la feuille de matière plastique (4) est une feuille multicouche dont le côté tourné vers le papier (6) se compose d'au moins un a) polymère d'éthylène et/ou copolymère d'éthylène et d'acétate de vinyle, de b) un homopolymère de styrène et/ou de c) un polymère du type caoutchouc,
a) étant présent en une proportion de 55 à 95 % en poids sous la forme d'un polymère polyéthylène ayant une masse volumique de 0,91 à 0,93 g/cm³ et un indice de fluidité à l'état fondu de 0,5 à 7,0 g/10 min. et le copolymère d'éthylène et d'acétate de vinyle contenant au maximum 10 % en poids d'acétate de vinyle, l'homopolymère de styrène b) étant présent en une proportion de 0 à 30 % en poids, et c) un copolymère séquencé thermoplastique élastomère styrène-butadiène-styrène ou styrène-isoprène-styrène étant présent en une proportion de 0 à 20 % en poids et/ou un polymère polybutylène ou polyisobutylène étant présent en une proportion de 0 à 30 % en poids,
le papier du fond de l'emballage est un papier traité par une matière plastique, qui présente une perméabilité moyenne aux gaz d'au moins 0,50 µm/Pa.s, et la feuille de matière plastique est scellée à la bande de papier.

2. Emballage selon la revendication 1, **caractérisé** en ce que la feuille de matière plastique multicouche (4) est obtenue par coextrusion.

3. Emballage selon la revendication 1, **caractérisé** en ce que la feuille de matière plastique multicouche (4) est un stratifié.

4. Emballage selon l'une quelconque des revendications 1 à 3, **caractérisé** en ce que le papier (6) présente un poids par unité de surface qui est compris entre 55 et 250 g/m².

5. Emballage selon l'une quelconque des revendications 1 à 4, **caractérisé** en ce que l'enduit de matière plastique présente un poids de 3 à 20 g/m².

6. Emballage selon l'une quelconque des revendications 1 à 5, **caractérisé** en ce que la matière plastique est un copolymère EVA.

7. Emballage selon l'une quelconque des revendications 1 à 6, **caractérisé** en ce que la matière plastique est appliquée d'un côté sur le papier (6).

8. Emballage selon l'une quelconque des revendications 1 à 7, **caractérisé** en ce que la matière plastique s'étend pratiquement sur toute l'épaisseur du papier.

9. Emballage selon l'une quelconque des revendications 1 à 8, **caractérisé** en ce que le papier (6) est constitué d'un type grossier de cellulose.

10. Emballage selon l'une quelconque des revendications 1 à 9, **caractérisé** en ce que la cellulose n'est pas broyée ou n'est que peu broyée.

11. Emballage selon l'une quelconque des revendications 1 à 10, **caractérisé** en ce que le papier (6) est un papier non chargé.

12. Emballage selon l'une quelconque des revendications 1 à 11, **caractérisé** en ce que le papier (6) a été renforcé par du silicate de sodium.

13. Emballage pour articles à stériliser, constitué d'une feuille de matière plastique formant une calotte, éventuellement emboutie, et d'une bande de papier formant le fond de l'emballage, la bande de papier étant scellée à la feuille de manière à être séparable, **caractérisé** en ce que la feuille de matière plastique (4) est éventuellement une feuille multicouche dont le côté tourné vers le papier (6) est constitué d'un polypropylène ou d'un copolymérisat de polypropylène, le papier (6) du fond de l'emballage est un papier traité par une matière plastique, qui présente une perméabilité moyenne aux gaz d'au moins 0,50 µm/Pa.s et qui possède un poids par unité de surface compris entre 55 et 250 g/m², l'enduit de matière plastique présente un poids de 3 à 20 g/m² , la matière plastique est un copolymère EVA qui est appliqué d'un côté sur le papier (6) et qui s'étend pratiquement sur toute l'épaisseur du papier.

## Claims

1. Pack for articles to be sterilized, comprising an optionally thermoformed plastic film forming a dome, and a paper web forming the pack base, where the paper web is heat-sealed to the film in a peelable manner, characterized in that the plastic film (4) is a multilayer film whose side facing the paper (6) is a copolymer comprising
a) at least one ethylene polymer and/or ethylene-vinyl acetate copolymer, and
b) a styrene homopolymer, and/or
c) an elastomeric polymer;
where
a) is present in an amount of 55-95 per cent by weight as a polyethylene polymer having a density of from 0.91 to 0.93 g/ccm and a melt flow index of from 0.5 to 7.0 g/10 min, and the ethylene-vinyl acetate copolymer contains a maximum of 10 per cent by weight of vinyl acetate;
b) the styrene homopolymer is present in an amount of from 0 to 30 per cent by weight;
c) an elastomeric, thermoplastic styrene-butadienestyrene block copolymer or styrene-isoprene-styrene block copolymer is present in an amount of from 0 to 20 per cent by weight and/or a polybutylene or polyisobutylene polymer is present in an amount of from 0 to 30 per cent by weight;
the paper of the pack base is plastic-treated paper having a mean gas permeability of at least 0.50 µm/Pa.s; and the plastic film is heat-sealed to the paper web.

2. Pack according to Claim 1, characterized in that the multilayer plastic film (4) is a coextrudate.

3. Pack according to Claim 1, characterized in that the multilayer plastic film (4) is a laminate.

4. Pack according to one of Claims 1 to 3, characterized in that the paper (6) has a basis weight of from 55 to 250 g/m².

5. Pack according to one of Claims 1 to 4, characterized in that the plastic coating has a weight of 3-20 g/m².

6. Pack according to one of Claims 1 to 5, characterized in that the plastic is an EVA copolymer.

7. Pack according to one of Claims 1 to 6, characterized in that the plastic has been applied to one side of the paper (6).

8. Pack according to one of Claims 1 to 7, characterized in that the plastic extends essentially over the entire thickness of the paper.

9. Pack according to one of Claims 1 to 8, characterized in that the paper (6) comprises a coarse grade of pulp.

10. Pack according to one of Claims 1 to 9, characterized in that the cellulose has not been beaten or has only been beaten insignificantly.

11. Pack according to one of Claims 1 to 10, characterized in that the paper (6) is an unfilled paper.

12. Pack according to one of Claims 1 to 11, characterized in that the paper (6) has been strengthened using sodium silicate.

13. Pack for articles to be sterilized, comprising an optionally thermoformed plastic film forming a dome, and a paper web forming the pack base, where the paper web has been heat-sealed to the film in a peelable manner, characterized in that the plastic film (4) is optionally a multilayer film whose side facing the paper (6) is a polypropylene or a polypropylene copolymer; the paper (6) of the pack base is a plastic-treated paper having a mean gas permeability of at least 0.50 µm/Pa.s and a basis weight of from 55 to 250 g/m², the plastic coating has a weight of 3-20 g/m², the plastic is an EVA copolymer which has been applied to one side of the paper (6) and extends essentially over the entire thickness of the paper.
